(19) Europäisches Patentamt
European Patent Office
Office européen des brevets

(11) Numéro de publication: **0 033 323**
**B1**

(12) # FASCICULE DE BREVET EUROPEEN

(45) Date de publication du fascicule du brevet: **01.08.84**

(51) Int. Cl.³: **C 07 D 307/50**, C 13 K 13/00

(21) Numéro de dépôt: **80901462.4**

(22) Date de dépôt: **31.07.80**

(86) Numéro de dépôt international: **PCT/FR 80/00128**

(87) Numéro de publication internationale: **WO 81/00407 (19.02.81 Gazette 81/5)**

(54) **PERFECTIONNEMENTS APPORTES AUX PROCEDES ET AUX APPAREILLAGES POUR L'OBTENTION DE FURFURAL A PARTIR DE MATIERES VEGETALES.**

(30) Priorité: **03.08.79 FR 7919935**

(43) Date de publication de la demande: **12.08.81 Bulletin 81/32**

(45) Mention de la délivrance du brevet: **01.08.84 Bulletin 84/31**

(84) Etats contractants désignés: **CH DE FR GB LI SE**

(56) Documents cités:
**DE - C - 719 704**
**DE - C - 740 602**
**FR - A - 720 424**
**FR - A - 1 024 196**
**GB - A - 922 685**

(73) Titulaire: **BERTIN & CIE, Boîte Postale No. 3, F-78370 Plaisir (FR)**

(72) Inventeur: **RAYMOND, Bernard Pierre Marie, Rue de Catoy, F-40440 Ondres (FR)**

(74) Mandataire: **Ores, Irène et al, CABINET ORES 6, Avenue de Messine, F-75008 Paris (FR)**

EP 0 033 323 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à un nouveau procédé de préparation du furfural à partir de matières végétales, et à l'appareillage pour la mise en œuvre de ce procédé.

Le furfural est un composé de formule:

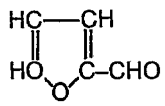

qui présente une grande importance industrielle en raison de ses nombreuses applications, notamment pour la fabrication de fibres textiles synthétiques, de matières plastiques et de caoutchoucs synthétiques.

Le furfural est obtenu à partir de matières végétales contenant des pentosanes, telles qu'épis de maïs, balles d'avoine, de riz ou de coton, par hydrolyse des pentosanes pour obtenir des pentoses, qui, par déshydratation, donnent le furfural, conformément aux réactions suivantes:

$$(C_5H_8O_4)_n + H_2O \rightarrow n\ C_5H_{10}O_5 \qquad (1)$$

pentosane pentose

$$_nC_5H_{10}O_5 \rightarrow n\ C_5H_4O_2 + 3n\ H_2O \qquad (2)$$

furfural

Il a été proposé de fabriquer le furfural à partir de matières végétales contenant des pentosanes par traitement de ces dernières, préalablement humectées d'un acide dilué non volatil, qui joue le rôle de catalyseur de la dégradation des pentosanes selon les réactions (1) et (2), par de la vapeur d'eau sous une pression relativement élevée, de l'ordre de 4,2 kg/cm². Suivant cette technique, décrite dans le brevet des Etats-Unis d'Amérique n° 1 735 084 du 7 septembre 1922, le traitement par la vapeur d'eau sous pression réalise en même temps la dégradation des pentosanes et la distillation du furfural résultant de cette dégradation. Toutefois, les rendements en furfural obtenus par ce procédé sont très faibles, en sorte que celui-ci ne présente qu'un faible intérêt économique pour la production de furfural à l'échelle industrielle. C'est pourquoi on a cherché à améliorer les procédés de production de furfural à partir de matières végétales contenant des pentosanes, en soumettant ces dernières à un traitement à la vapeur d'eau comportant essentiellement deux étapes, comme décrit dans le brevet français 1 181 953 du 3 septembre 1957. Suivant le procédé décrit dans ce brevet, la matière première est soumise à un traitement préalable par la vapeur d'eau, sous une pression voisine de la pression atmosphérique, puis à une deuxième étape de traitement par la vapeur d'eau sous une pression plus élevée, variant de 2,8 kg/cm² en début d'opération à 7 kg/cm² en fin d'opération. L'acide non volatil utilisé comme catalyseur peut être de l'acide sulfurique, de l'acide phosphorique ou un acide alcane-sulfonique et peut être ajouté aux matières végétales indifféremment soit lors de la deuxième étape du traitement, soit lors de l'étape de traitement préalable. La vapeur d'eau mise en œuvre est de la vapeur d'eau surchauffée à 270 °C environ sous une pression de 10,5 kg/cm² et le rendement en furfural mentionné est de 68% de la théorie, ce qui devrait correspondre à 16% environ en poids de furfural par rapport au poids sec de la charge. La première étape du procédé peut se dérouler dans un récipient de construction moins robuste que le digesteur, qui doit être apte à supporter les températures et les pressions élevées, dans lequel se déroule la deuxième étape du procédé. Ce procédé présente cependant lui aussi de nombreux inconvénients, représentés par: – les rendements encore insuffisants en furfural qu'il assure et – la mise en œuvre de températures et de pressions de vapeur élevées, qui nécessite l'utilisation, au moins au cours de la deuxième étape du procédé, d'appareillages spéciaux, en règle générale très coûteux, aptes à supporter de telles températures et pressions, et présentant des risques d'explosion.

Il existe également un procédé dit «Procédé Agrifurane» (cf. «Techniques de l'ingénieur – Génie chimique», Vol. 4, page J. 6020–1501) de production du furfural par hydrolyse en milieu acide de matières végétales riches en pentosanes. Ce procédé réalise l'hydrolyse par injection de vapeur dans des réacteurs en acier, sous une pression de 10 bars. Les vapeurs furfuralées qui sortent de ces réacteurs contiennent 5 à 6% en poids de furfural, en sorte qu'il est nécessaire, pour récupérer le furfural technique à 90%, non seulement de les condenser, mais de les soumettre à une distillation azéotropique, qui est une opération relativement compliquée et coûteuse. Le rendement en furfural obtenu est de 10 à 13% par rapport au poids sec de la matière première mise en œuvre. Ce procédé présente donc l'inconvénient de mettre en œuvre des pressions élevées, qui nécessitent l'utilisation de réacteurs résistant à ces pressions, et le rendement en furfural qu'il permet d'obtenir est extrêmement faible et ne peut être atteint qu'au prix de traitements d'élimination de l'eau qui impliquent des appareillages relativement onéreux et qui sont longs et compliqués.

D'autres techniques de fabrication du furfural ont été proposées. En particulier, les brevets allemands Henkel 719 704 et Chemische Fabrik Löwenberg Dr. Warth & Co 740 602 produisent du furfural en deux étapes mais avec de l'acide sulfurique dilué, donc à des températures élevées et sous une pression élevée, ce qui se traduit par des dégradations du furfural et donc par une réduction du rendement, tandis que l'appareillage nécessaire est onéreux du fait de la nécessité de mettre en œuvre des températures et des pressions élevées.

Le brevet français Blomqvist & Groth 720 424 utilise pour l'hydrolyse des matières végétales, un acide dilué qu'il est impossible de récupérer et de recycler et, en raison de la mise en œuvre de l'acide dilué, il applique des températures élevées qui provoquent la résinification du furfural et réduisent de ce fait le rendement en furfural.

De plus, la solution de furfural obtenue en sortie

de furfuralisation n'est que de 1% de furfural, ce qui nécessite des moyens de séparation supplémentaires très coûteux en investissements et/ou en consommation d'énergie.

Le brevet britannique Ledoga 922 685 réalise l'hydrolyse avec un acide très concentré, puisqu'il est à plus de 20% en poids par rapport à la matière végétale, et non par rapport au poids de liquide, ce qui le rend difficile à régénérer, sa régénération ne pouvant se faire qu'au prix d'investissements et de consommation d'énergie très élevés et en tout cas incompatibles avec une production industrielle.

D'autre part, le procédé selon ce brevet réalise uniquement l'hydrolyse pour produire des xyloses et ne cherche pas à obtenir du furfural.

La présente invention s'est en conséquence donné pour but de pourvoir à un procédé perfectionné de production de furfural, qui répond mieux aux nécessités de la pratique que les procédés proposés conformément à l'Art antérieur, notamment en ce qu'il est plus économique que les procédés de l'Art antérieur, car il permet la production de furfural sans avoir recours à l'application de températures et de pressions élevées, en ce qu'il permet la récupération et le recyclage de l'acide utilisé comme catalyseur, en ce qu'il permet d'améliorer les rendements de production de furfural et en ce qu'il peut être mis en œuvre dans des appareillages relativement peu onéreux, qui n'ont pas à subir des contraintes imposées par des températures et des pressions élevées.

La présente invention a pour objet un procédé de production de furfural à partir de matières végétales contenant des pentosanes, par hydrolyse de ces derniers en présence d'un acide fort, et déshydratation subséquente, lequel procédé est caractérisé en ce qu'il est réalisé en deux étapes dont chacune est menée dans un réacteur séparé, en ce qu'au cours d'une première étape, on effectue, dans un premier réacteur, l'hydrolyse des pentosanes contenus dans la matière végétale, en présence d'acide chlorhydrique 5 à 6N à 20% en poids de HCL, l'hydrolyse étant réalisée en l'espace de 1 à 2 heures, par circulation à contre-courant liquide-solide, à la pression atmosphérique, à une température de 20 à 70 °C, la proportion d'acide chlorhydrique représentant 66 à 108% en poids des matières végétales sèches, pour obtenir une solution de pentoses, et en ce qu'au cours d'une deuxième étape, qui est réalisée dans un deuxième réacteur, ladite solution de pentoses est déshydratée pour obtenir du furfural, par action de la vapeur à une pression de 1 à 2 bars absolus et à une température inférieure ou égale à 110 °C, en milieu acide chlorhydrique à 20% en poids, ladite déshydratation étant réalisée par circulation à contre-courant de la solution de pentoses et de la vapeur de déshydratation, la solution de pentoses étant admise en tête de réacteur d'où elle s'écoule par gravité alors que la vapeur circule à contre-courant à partir du fond du réacteur, pour obtenir séparément, en tête du réacteur, le furfural formé, qui fournit, après décantation, une couche de furfural à 90% en poids exempt d'acide et, en soutirage du réacteur, la solution d'acide chlorhydrique, qui est récupérée, pour être recyclée dans une nouvelle mise en œuvre du procédé, dans l'étape d'hydrolyse.

Conformément à l'invention, le furfural obtenu est soumis à un processus de purification approprié, pour obtenir du furfural pur.

Lorsque la durée de l'hydrolyse est limitée à 1 à 2 heures, seuls les pentosanes sont dégradés; si elle est prolongée au-delà de ces durées, la cellulose de la matière végétale est attaquée par l'acide fort concentré, pour donner lieu à des sucres, et notamment à du glucose.

Selon une modalité particulièrement avantageuse du procédé objet de l'invention, le processus d'hydrolyse est accéléré en soumettant le milieu réactionnel à agitation, en faisant recirculer la solution acide de pentoses obtenue au cours de la première étape, dans le milieu réactionnel constitué par les matières végétales et l'acide fort concentré.

Selon un autre mode de réalisation avantageux du procédé objet de l'invention, la vapeur de déshydratation mise en œuvre au cours de la deuxième étape du procédé est à une température de 100 à 110 °C environ.

Selon une disposition particulièrement avantageuse de l'invention, la solution de pentoses à déshydrater et la vapeur de déshydratation sont introduites en continu dans l'étape de déshydratation.

Selon une autre disposition particulièrement avantageuse de l'invention, la solution de pentoses à déshydrater est admise en tête du réacteur d'où elle s'écoule par gravité, alors que la vapeur circule à contre-courant à partir du fond du réacteur, permettant ainsi une extraction rapide en continu, du furfural formé, et évitant, par suite, toute réaction de résinification de ce dernier.

Selon encore une autre disposition avantageuse de l'invention, la solution de pentoses à déshydrater est additionnée d'un agent antimoussant approprié, qui, selon une modalité avantageuse de l'invention, peut être un antimoussant à base de silicones.

Selon une autre disposition avantageuse de l'invention, à la fin de l'étape de déshydratation, l'acide concentré est récupéré par simple décantation, pour être recyclé dans l'étape d'hydrolyse.

Selon une modalité avantageuse de l'invention, l'acide récupéré est soumis, préalablement à son recyclage, à un processus de distillation pour le ramener à sa composition azéotropique de 20% en poids.

Selon encore une autre disposition avantageuse de l'invention, les résidus résultant de l'hydrolyse des matières végétales sont séparés par simple chauffage, de l'acide concentré utilisé pour l'hydrolyse, qu'ils contiennent, si celui-ci est un acide volatil, pour être récupérés et valorisés.

La présente invention a également pour objet un appareillage pour la production de furfural et mettant en œuvre le procédé défini plus haut, lequel appareillage est caractérisé en ce qu'il comprend en combinaison: – au moins un premier

réacteur associé à un dispositif d'amenée des matières végétales et à un réservoir de stockage d'acide fort concentré auquel il est relié par une conduite d'alimentation qui amène l'acide fort concentré dans le premier réacteur à contre-courant de la matière végétale; – au moins un deuxième réacteur à la partie supérieure duquel débouche une conduite d'amenée de la solution de pentoses contenant l'acide fot concentré en provenance d'au moins un premier réacteur, et qui comporte à sa partie inférieure des moyens d'introduction de vapeur d'eau à une température de l'ordre de 100 à 110 °C pour assurer la circulation de la vapeur d'eau à contre-courant de la solution de pentosanes susdite; – une conduite d'évacuation de l'acide concentré sensiblement à la base dudit deuxième réacteur, laquelle conduite débouche dans la conduite d'alimentation en acide d'au moins un premier réacteur et qui recycle ledit acide dans ce dernier, et une conduite, raccordée à la tête dudit deuxième réacteur, d'évacuation des vapeurs furfuralées exemptes d'acide vers un condenseur et un décanteur d'où le furfural est envoyé par des moyens appropriés à un réservoir de stockage.

Selon un mode de réalisation avantageux de l'appareillage conforme à l'invention, au moins un premier réacteur est associé à des moyens d'agitation du mélange réactionnel qu'il contient et qui est essentiellement constitué par les matières végétales et l'acide fort concentré.

Selon une disposition avantageuse de ce mode de réalisation, les moyens d'agitation susdits sont essentiellement constitués par des moyens de recirculation de la solution acide de pentoses, qui recyclent cette dernière dans au moins un premier réacteur.

L'introduction des matières végétales dans un tel premier réacteur, ainsi que l'évacuation de leurs résidus, peuvent être effectuées de manière continue ou semi-continue par des orifices à sas de types connus.

Selon un autre mode de réalisation avantageux de l'appareillage conforme à l'invention, le(s) deuxième(s) réacteur(s) est (ou sont) rempli(s) d'un garnissage pour améliorer la distribution et le contact des réactifs dans le(s)dit(s) deuxième(s) réacteur(s).

Selon une disposition avantageuse de l'invention, le(s) deuxième(s) réacteur(s) est (ou sont) entouré(s) de moyens de réchauffage dudit (ou desdits) réacteur(s) tels que serpentins de réchauffage à la vapeur par exemple, pour limiter au maximum la condensation de la vapeur de déshydratation.

Selon une modalité avantageuse de l'invention, l'eau condensée récupérée à la sortie du condenseur et du décanteur, est envoyée à un bouilleur d'où elle est recyclée, après vaporisation, dans la conduite d'introduction de vapeur d'eau dans au moins un deuxième réacteur.

Conformément à l'invention, le bouilleur est, de préférence, un bouilleur fonctionnant en thermosiphon.

Conformément à l'invention, l'appareillage comporte en outre une installation de purification du furfural provenant du réservoir de stockage susdit, dans laquelle le furfural est débarrassé de l'eau qu'il contient et qui est reliée, par l'intermédiaire d'une conduite, à un réservoir de stockage de furfural anhydre.

Egalement conformément à l'invention, l'acide de déshydratation évacué du ou des deuxième(s) réacteur(s) est envoyé, préalablement à son recyclage dans au moins un premier réacteur, dans une colonne de distillation pour en extraire l'eau (notamment l'eau générée au cours de la réaction) et le ramener à sa composition initiale.

Selon une disposition avantageuse de l'invention, l'appareillage comprend, à la sortie d'au moins un premier réacteur, un condenseur-évaporateur dans lequel les sucres, et en particulier le glucose, obtenus par attaque acide de la cellulose des matières végétales, sont séparés de l'acide évacué d'au moins un deuxième réacteur, pour être récupérés à la sortie dudit condenseur-évaporateur, préalablement au recyclage de l'acide dans au moins un premier réacteur.

Pour la mise en œuvre du procédé de production de furfural conforme à la présente invention, l'on opère de préférence dans les conditions exposées ci-après:

La matière première contenant des pentosanes mise en œuvre, est constituée par différents déchets végétaux, tels que rafles de maïs, balles d'avoine, de riz, de coton ou autres résidus d'origine agricole, ou par des matières végétales à structure rigide, comme des souches de bois, ou encore par des déchets de scieries tels que chutes de scierie, copeaux, sciure, poussière de bois.

L'acide fort concentré mis en œuvre dans le procédé est de l'acide chlorhydrique 6-6N.

La vapeur d'eau mise en œuvre dans la deuxième étape du procédé, à savoir l'étape de déshydratation des pentoses obtenus dans la première étape du procédé, est de la vapeur d'eau à 100–110 °C, à une pression de 1 à 2 bars, ce qui procure un certain nombre d'avantages, qui seront explicités plus loin.

Le procédé qui fait l'objet de la présente invention est mis en œuvre suivant le schéma de principe représenté à la fig. 1 des dessins annexés.

On introduit dans un premier réacteur les matières végétales à traiter ainsi que l'acide chlorhydrique à une température comprise entre 20 et 70 °C et de préférence comprise entre 30 et 60 °C. La réaction d'hydrolyse acide des pentosanes des matières végétales, est réalisée en l'espace de 1 à 2 heures. Si l'on prolonge la durée de contact entre l'acide et les matières végétales, la cellulose de ces dernières est alors attaquée par l'acide, et est dégradée pour donner des sucres, et en particulier du glucose, qui sont récupérés, par séparation de l'acide dans une installation appropriée, préalablement au recyclage de l'acide dans le premier réacteur: il a été constaté que l'on obtient un bon rendement en glucose pour une durée moyenne de contact entre l'acide et les matières végétales, de l'ordre de 8 heures.

L'hydrolyse des pentosanes donne lieu à une

solution de pentoses qui est envoyée dans un deuxième réacteur, éventuellement après passage dans un échangeur de chaleur, tandis que les résidus de l'hydrolyse sont récupérés à leur sortie du premier réacteur, pour être valorisés, après avoir été traités par chauffage pour les débarrasser de l'acide volatil qui se sépare en entraînant avec lui l'eau éventuellement contenue dans les matières végétales. Ces résidus peuvent être utilisés soit comme combustibles, par exemple pour produire la vapeur nécessaire à la deuxième étape du procédé, soit comme source de protéines végétales.

Le deuxième réacteur est avantageusement constitué par une colonne remplie d'un garnissage, en céramique par exemple, qui favorise le contact et la distribution des réactifs dans ledit réacteur.

Dans certains cas cependant, on observe un bouchage progressif du garnissage de ce deuxième réacteur dans lequel a lieu la déshydratation par des impuretés entraînées issues des matières végétales à traiter. En pareil cas, on peut filtrer la solution de pentoses à l'aide d'un filtre monté sur la conduite d'alimentation de ce deuxième réacteur. En variante, on peut remplacer la colonne à garnissage par plusieurs, trois par exemple, deuxièmes réacteurs agités, montés en série.

A sa sortie du premier réacteur, la solution de pentoses contenant de l'acide concentré est envoyée à la partie supérieure du deuxième réacteur, tandis que la vapeur d'eau à 100–110 °C est introduite, à une pression de 1 à 2 bars, et, de préférence, à la pression atmosphérique, à la base dudit deuxième réacteur. Les vapeurs contenant du furfural se dégagent du deuxième réacteur pour être amenées à un condenseur relié à la partie supérieure dudit deuxième réacteur. Les vapeurs furfuralées obtenues à la sortie du deuxième réacteur contiennent 30% de furfural, ce qui constitue un avantage important: en effet, du fait que l'on effectue la réaction de furfuralisation à la pression atmosphérique, les vapeurs qui sortent du réacteur de furfuralisation sont plus chargées en furfural qu'elles ne peuvent l'être dans un traitement de furfuralisation qui utilise une pression de l'ordre de 10 bars, comme c'est le cas dans le procédé Agrifurane. Cette teneur de l'ordre de 30% en furfural dans les vapeurs, permet de récupérer le furfural par une simple opération de condensation et de décantation et ne nécessite pas le recours à un traitement de distillation azéotropique, de mise en œuvre difficile, avec un appareillage onéreux.

Après passage dans le condenseur et dans un décanteur approprié, l'eau condensée est envoyée dans un bouilleur, qui fonctionne de préférence en thermo-siphon, dans lequel elle est vaporisée, puis recyclée dans la conduite d'admission de vapeur d'eau qui alimente le deuxième réacteur en vapeur d'eau.

A sa sortie du décanteur, le furfural est récupéré dans un réservoir de stockage sous forme de furfural technique à 90%, après avoir barboté, le cas échéant, dans un neutraliseur contenant du carbonate de soude et de la soude pour en éliminer les traces d'acide qu'il contient.

Bien que l'on ait fait référence dans ce qui précède, à un premier réacteur dans lequel a lieu l'hydrolyse des pentosanes en pentoses, et à un deuxième réacteur dans lequel a lieu la déshydratation des pentoses en furfural, il va de soi que l'installation industrielle peut comprendre une pluralité de premiers réacteurs montés en série et/ou en parallèle et une batterie de deuxièmes réacteurs qui sont montés en série et/ou en parallèle et dans laquelle la solution de pentoses et la vapeur d'eau circulent successivement à contre-courant, pour améliorer la distribution et le contact vapeur/solution de pentoses et parfaire la réaction de déshydratation des pentoses en furfural.

Le furfural technique à 90% peut avantageusement être soumis à un traitement complémentaire de purification pour le débarrasser de l'eau qu'il contient encore à la fin du traitement de déshydratation, par distillation dans une colonne d'épuration, telle que colonne à plateaux sous vide, d'où le furfural anhydre est récupéré pour être envoyé dans un réservoir de stockage, tandis que l'eau est récupérée et débarrassée, dans un condenseur et un séparateur, des traces de furfural qu'elle contient, qui sont renvoyées à la colonne de distillation, tandis que l'eau peut être récupérée par tous moyens appropriés pour être éventuellement recyclée, après vaporisation, dans la conduite d'admission de vapeur d'eau dans le deuxième réacteur.

Le procédé qui fait l'objet de la présente invention présente de nombreux avantages par rapport aux procédés proposés dans l'Art antérieur.

En effet, la réalisation du procédé dans deux réacteurs séparés, en utilisant des conditions de température modérées et sensiblement la pression atmosphérique, minimise les réactions secondaires qui se produisent dans les procédés de l'Art antérieur au stade de la réaction de déshydratation, et notamment la résinification du furfural lors de sa formation, les réactions de condensation du furfural, l'oxydation du furfural par l'oxygène contenu dans les matières végétales, le risque de ces réactions secondaires étant pratiquement éliminé non seulement par l'utilisation des conditions de température et de pression objets de l'invention, mais aussi par le fait que la déshydratation des pentoses a lieu dans une enceinte distincte d'où les matières végétales initialement traitées sont absentes et que, de plus, le furfural peut être séparé de la phase liquide, sous forme de vapeurs furfuralées, au fur et à mesure de sa formation, évitant ainsi les réactions parasites résultant d'un contact prolongé entre le furfural et la phase liquide.

L'utilisation, pour l'hydrolyse acide, d'acide chlorhydrique 5N à 6N, permet d'obtenir un bon rendement d'hydrolyse: elle réduit considérablement la durée de la réaction; de plus, lors de l'étape de déshydratation, l'acide n'est pas entraîné par la vapeur et peut ainsi être recyclé vers

le réacteur d'hydrolyse après séparation éventuelle des sucres de l'acide. Cet avantage supplémentaire est dû au fait que la déshydratation est réalisée à 100–110 °C, c'est-à-dire à une température inférieure à la température de vaporisation de l'azéotrope eau-acide chlorhydrique (la température de vaporisation de l'azéotrope eau-HCl est à 110 °C), et que l'on a une concentration en acide proche de l'azéotrope: 20% en poids d'HCl. A cela vient s'ajouter, comme on l'a dit plus haut, que l'utilisation de l'acide chlorhydrique, facilite considérablement la récupération et la valorisation des résidus obtenus après hydrolyse car cet acide se sépare très aisément des résidus solides par chauffage, en entraînant l'eau éventuellement contenue dans les matières végétales traitées.

La possibilité de recycler l'acide sortant du ou des réacteurs de déshydratation, représente un facteur économique très favorable dans la mise en œuvre du procédé conforme à l'invention.

En outre, la suppression quasi-totale des réactions parasites améliore de façon importante, par voie de conséquence, le rendement en furfural obtenu.

D'autre part, la souplesse du procédé conforme à l'invention, dans lequel on peut choisir à volonté la durée de la réaction d'hydrolyse acide, permet de contrôler la composition de l'hydrolysat obtenu, et en particulier d'obtenir soit uniquement des pentoses, soit également d'autres produits valorisables tels que des sucres.

Un autre avantage du procédé objet de l'invention réside dans le fait que l'utilisation de températures modérées et de la pression atmosphérique permet le déroulement du procédé conforme à l'invention dans un appareillage relativement peu coûteux, attendu qu'il n'a pas à être soumis aux contraintes résultant de l'utilisation de températures et de pressions élevées comme c'est le cas dans l'Art antérieur. De plus, les investissements énergétiques sont considérablement réduits par rapport aux procédés de l'Art antérieur du fait que l'on travaille à des températures relativement basses et à la pression atmosphérique, éliminant en outre les risques de pertes thermiques du fait des conditions opératoires particulièrement favorables du procédé.

Par ailleurs, la sécurité des installations est considérablement améliorée par rapport à l'Art antérieur et, en particulier, les risques d'explosion sont éliminés du fait que l'on travaille à la pression atmosphérique.

D'autre part, les conditions de mise en œuvre du procédé conforme à l'invention permettent une marche en continu, au stade de la déshydratation, réduisant ainsi la durée totale du procédé de production de furfural par rapport aux durées nécessaires dans les procédés de l'Art antérieur.

Un autre avantage important du procédé objet de l'invention, est d'éliminer la nécessité de la distillation azéotropique nécessaire dans les procédés de l'Art antérieur, puisque les vapeurs furfuralées qui sortent de l'étape de furfuralisation contiennent environ 30% de furfural.

Outre les dispositions qui précèdent, l'invention comprend encore d'autres dispositions, qui ressortiront de la description qui va suivre.

L'invention sera mieux comprise à l'aide du complément de description qui va suivre, qui se réfère aux dessins annexés dans lesquels:

– la fig. 1 représente, comme déjà mentionné, le schéma de principe du procédé conforme à l'invention;

– la fig. 2 représente de façon schématique, un mode de réalisation d'une installation de production de furfural, conforme à l'invention, laquelle se réfère, en outre, à un exemple de mise en œuvre du procédé dans un appareillage conforme à l'invention;

– la fig. 3 représente, de façon schématique, un autre mode de réalisation d'une installation de production de furfural, conforme à l'invention, et

– la fig. 4 représente sous forme de courbes l'influence de la température sur la cinétique de la réaction d'hydrolyse, tandis que

– la fig. 5 représente les courbes des rendements molaires de la réaction de furfuralisation.

La fig. 1 représente un schéma de principe du procédé qui fait l'objet de l'invention, dans lequel les matières végétales sont introduites par un dispositif approprié 1, dans un premier réacteur 2 dans lequel un acide fort concentré est également introduit par une conduite 3. L'acide fort concentré, qui est avantageusement de l'acide chlorhydrique 5,5N, à 20% de concentration, est introduit dans le réacteur 2, à une température comprise entre 20 et 70 °C, et de préférence, entre 30 et 60 °C. L'hydrolyse a lieu dans le réacteur 2, à la pression atmosphérique.

La solution de pentoses obtenue par hydrolyse des pentosanes contenus dans les matières végétales traitées en présence de l'acide, est envoyée par la conduite 4 dans un deuxième réacteur 5, dans lequel elle parvient, éventuellement après réchauffage dans un réchauffeur 6. Alors que la solution de pentoses contenant l'acide fort concentré est introduite à la partie supérieure du réacteur 5, la vapeur d'eau à 100–110 °C est introduite à la base du réacteur 5, en sorte qu'il s'établit une circulation à contre-courant entre la solution de pentoses à déshydrater et la vapeur d'eau à 100–110 °C. La réaction de déshydratation des pentoses a lieu sensiblement à la pression atmosphérique à la température de la vapeur d'eau, c'est-à-dire à 100–110 °C.

Le furfural est évacué du réacteur 5, de préférence à la partie supérieure de ce dernier, sous forme de vapeurs furfuralées qui sont envoyées dans un condenseur, tandis que l'acide qui constitue la phase liquide, est soutiré à la base du réacteur 5, pour être recyclé, éventuellement après refroidissement dans l'échangeur 6, dans le réacteur 2 d'hydrolyse.

Les résidus végétaux obtenus à la suite de l'hydrolyse des matières végétales, sont soutirés du réacteur 2 pour subir un traitement thermique d'élimination de l'acide résiduel qu'ils contiennent, en vue de leur récupération et de leur valorisation.

L'installation de production de furfural représentée à titre d'exemple non limitatif à la fig. 2 est conçue selon le principe du schéma de fonctionnement représenté à la fig. 1.

Cette installation comprend trois unités:

- une unité dans laquelle est réalisée l'hydrolyse des pentosanes en pentoses;
- une unité dans laquelle les pentoses sont déshydratés pour donner du furfural;
- une unité d'épuration du furfural technique obtenu lors de la deuxième étape, pour obtenir du furfural anhydre.

L'unité d'hydrolyse comprend deux réacteurs 10 dans chacun desquels les matières végétales à traiter sont introduites à l'aide d'un dispositif approprié, tel qu'un transporteur à courroie 11 qui coopère avec un élévateur 12 qui transfère les matières végétales de leur lieu de stockage 13 au transporteur 11. Les matières végétales sont avantageusement introduites par gravité dans les réacteurs 10.

L'acide fort concentré provenant d'un réservoir de stockage 14 est introduit à une température de 20 à 70 °C, et de préférence à une température de 30 à 60 °C, dans les réacteurs 10, par la conduite 15. La réaction d'hydrolyse acide des pentosanes contenus dans les matières végétales a lieu dans les réacteurs 10, à la pression atmosphérique et à une température modérée avantageusement comprise entre 20 et 70 °C, et de préférence comprise entre 30 et 60 °C.

Du fait de l'utilisation d'un acide fort concentré, la réaction d'hydrolyse est extrêmement efficace et rapide; sa durée est de 1 à 2 heures en moyenne. Toutefois, dans la mesure où l'on souhaite obtenir non seulement du furfural, mais également des sucres résultant de l'attaque de la cellulose des matières végétales par l'acide, on peut prolonger la durée de la réaction dans les réacteurs 10. Il est apparu qu'un temps de contact entre les matières végétales et l'acide de l'ordre de 8 heures, permet d'obtenir une dégradation de la cellulose jusqu'au glucose, lequel est récupéré ainsi qu'il sera précisé plus loin. La durée de la réaction d'hydrolyse est également déterminée par le type de matières végétales traitées: c'est ainsi que sa durée sera plus longue dans le cas où l'on traite des matières végétales à structure rigide, telles que des souches de bois par exemple.

On recueille à la sortie des réacteurs 10, d'une part les résidus végétaux de l'hydrolyse qui sont soutirés par les conduites 16 et, d'autre part, la solution de pentoses contenant l'acide fort concentré. Dans le cas où l'on réalise non seulement l'hydrolyse des pentosanes, mais aussi la dégradation de la cellulose en sucres, et notamment en glucose, il est nécessaire de prévoir un appareillage de séparation des sucres de l'acide d'attaque, lequel appareillage peut avantageusement être constitué par un évaporateur-condenseur 32 monté en dérivation sur la conduite 42, à la sortie duquel les sucres, notamment le glucose, sont récupérés (en 33), tandis que l'acide est recyclé par la conduite 34, dans la conduite 15 d'alimentation des réacteurs 10 en acide.

La réaction d'hydrolyse est accélérée dans les réacteurs 10 en soumettant le milieu réactionnel, constitué par les matières végétales et l'acide, à agitation. Cette agitation est avantageusement obtenue par recirculation de la solution acide de pentoses obtenue dans le milieu réactionnel: tout ou partie de ladite solution est repris par une pompe 46 et recyclé dans chacun des réacteurs 10 par une conduite 47.

La solution de pentoses contenant l'acide fort concentré est envoyée, éventuellement après mise en température dans un échangeur de chaleur 43, par une conduite 17 dans une batterie de colonnes 18, 19, 20 montées en série, contenant un garnissage de contact 21, en céramique par exemple.

De la vapeur d'eau à 100–110 °C est également envoyée par une conduite 22 dans la batterie de colonnes 18, 19, 20. Alors que la solution de pentoses est introduite successivement à la partie supérieure des colonnes 18, 19, 20 par les conduites 17, 23 et 24, la vapeur à 100–110 °C est introduite à la pression atmosphérique à la base de la colonne 20, dans laquelle elle circule à contre-courant de la solution de pentoses; elle transforme ainsi par déshydratation les pentoses en furfural qu'elle entraîne lors de son passage successivement dans les colonnes, soit 19 par la conduite 25, et 18 par la conduite 26: les vapeurs furfuralées sont évacuées au sommet de la colonne 18 par une conduite 27; ces vapeurs furfuralées contiennent 30% de furfural du fait que le traitement s'est déroulé à la pression atmosphérique, permettant ainsi aux vapeurs de recevoir une charge plus importante en furfural que ce n'est le cas dans les installations de traitement de l'Art antérieur, dans lesquelles la pression appliquée, qui est généralement de l'ordre de 10 bars, empêche les vapeurs de se charger en furfural dans une proportion supérieure à 5 à 6%. Du fait de leur teneur élevée en furfural, les vapeurs furfuralées n'ont pas à subir, pour permettre la récupération du furfural, un traitement de distillation azéotropique comme c'est le cas dans l'Art antérieur; un simple traitement par passage dans un condenseur 28, puis un décanteur 29, suffit pour récupérer le furfural liquide; le furfural liquide, décanté dans le décanteur 29, est envoyé à un réservoir de stockage 30 éventuellement après neutralisation, l'eau condensée étant envoyée du décanteur 29 dans un bouilleur 31 où elle est vaporisée pour être recyclée dans la conduite 22 d'alimentation de la colonne 20 en vapeur d'eau à 100–110 °C. L'acide chlorhydrique est soutiré à la base de la colonne 20 d'où il est recyclé par la conduite 42 dans la conduite 15 d'alimentation des réacteurs 10 en acide, après avoir été refroidi à une température de 20–70 °C et de préférence à 30–60 °C dans un échangeur de chaleur 43 et après avoir été débarrassé des sucres qu'il contient, dans l'évaporateur-condenseur 32, comme indiqué plus haut.

Le furfural recueilli dans le réservoir 30 est un furfural technique à 90% d'où il est nécessaire d'éliminer l'eau présente en tant qu'impureté,

dans la mesure où l'on désire obtenir du furfural anhydre. Cette étape d'épuration est réalisée par circulation de la solution de furfural technique à 90% dans une installation d'épuration connue en elle-même, telle qu'une colonne de distillation à plateaux 36. Le furfural est introduit par la conduite 35 dans ladite colonne à 50 °C environ, sous un vide de 0,1 bar. Le furfural anhydre obtenu est soutiré par une conduite 37 en direction de réservoirs de stockage 44, tandis que l'eau séparée du furfural et contenant une faible quantité de furfural dissous est envoyée dans un condenseur 38, puis un séparateur 39 d'où le furfural décanté est renvoyé dans la colonne 36 par une conduite 40, tandis que l'eau est évacuée, par une conduite 45, vers un éjecteur 41 qui l'envoie, au besoin, par tous moyens appropriés au bouilleur 31 où elle est vaporisée, puis recyclée dans la conduite 22.

Exemple de mise en œuvre du procédé de production de furfural conforme à l'invention

Des rafles de maïs à 12% d'humidité sont introduites à raison de 1,95 tonne/heure dans chacun des réacteurs 10, par l'intermédiaire du transporteur à courroie 11. De l'acide chlorhydrique 5,5N à 20% de concentration azéotropique et à une température de 40 °C, provenant du réservoir 14, est introduit par la conduite 15 dans chacun des réacteurs 10.

La durée de l'hydrolyse acide, qui est réalisée dans les réacteurs 10 à une pression de 1 bar et à une température de 40 °C, est de 8 heures environ. La durée de l'hydrolyse des pentosanes contenus dans les matières végétales, en pentoses n'est que de 1 heure, dans les conditions de la réaction; la prolongation de la durée de contact des matières végétales avec l'acide chlorhydrique utilisé comme catalyseur de l'hydrolyse, jusqu'à 8 heures, provoque l'attaque de la cellulose des matières végétales et sa dégradation jusqu'au stade du glucose. La solution de pentoses obtenue qui contient l'acide chlorhydrique et le glucose est recyclée au moins en partie à l'aide de la pompe 46 et de la conduite 47 dans chacun des réacteurs 10, pour provoquer une agitation dans le réacteur et accélérer d'une part l'hydrolyse et, d'autre part, la dégradation de la cellulose. La solution de pentoses qui contient l'acide chlorhydrique, quitte les réacteurs 10 par la conduite 17, vers l'étape de furfuralisation. Les résidus végétaux de l'hydrolyse sont soutirés des réacteurs 10 par la conduite 16, à raison de 1,45 tonne/heure, vers des unités de traitement appropriées.

La solution acide de pentoses est envoyée, par la conduite 17, dans une batterie de colonnes garnies 18, 19, 20 en même temps que de la vapeur d'eau à 105 °C et à une pression de 1 à 1,3 bars, est introduite à contre-courant dans la batterie de colonnes 20, 19, 18.

A leur sortie de la batterie de colonnes 18, 19, 20, les vapeurs furfuralées à 100 °C, contenant 30% de furfural, sont traitées dans un condenseur, puis dans un décanteur pour récupérer du furfural technique à 90% et de l'eau qui est vaporisée et recyclée sous forme de vapeur dans la batterie de colonnes 20, 19, 18.

L'acide chlorhydrique est soutiré, à raison de 13 $m^3$/heure, à la base de la colonne 20 pour être recyclé dans le circuit 15 d'alimentation en acide des réacteurs 10, éventuellement après remise à une température de 40 °C dans l'échangeur 43. Avant d'être recyclé dans le circuit 15 d'alimentation en acide des réacteurs 10, l'acide chlorhydrique de la conduite 42 est envoyé, par une dérivation, dans un évaporateur-condenseur 32 dans lequel il est débarrassé du glucose qu'il contient, lequel est récupéré en 33, en vue de son utilisation et/ou de sa valorisation éventuelle.

L'on obtient du furfural anhydre à raison de 250 kg/heure, par traitement du furfural technique à 90% obtenu à l'issue du stade de déshydratation, dans une installation d'épuration sous vide (0,1 bar) à 50 °C comprenant essentiellement une colonne de distillation à plateaux ou autres, comme décrit plus haut en relation avec la fig. 2.

L'installation de production de furfural représentée à titre d'exemple non limitatif à la fig. 3 est conçue, de même que l'installation représentée à la fig. 2, selon le principe du schéma de fonctionnement représenté à la fig. 1.

Dans l'installation de la fig. 3, l'unité dans laquelle est réalisée l'hydrolyse des pentosanes en pentoses comprend un seul réacteur 48, dans lequel les matières végétales à traiter sont introduites en continu à l'aide d'un dispositif approprié tel que celui décrit en référence à la fig. 2.

L'acide fort concentré provenant d'un réservoir de stockage 49 est introduit en continu à une température de 60 °C, dans le réacteur 48 par la conduite 50, à contre-courant des matières végétales. La réaction d'hydrolyse acide des pentosanes contenus dans les matières végétales a lieu dans le réacteur 48, à la pression atmosphérique et à une température modérée, de l'ordre de 60 °C, en l'espace de 1 à 2 heures. Les résidus végétaux de l'hydrolyse, sont recueillis, à la base du réacteur 48, par l'intermédiaire d'un sas réactif 87 par exemple, sur un filtre à bande 51, où ils subissent un égouttage, et d'où ils sont envoyés dans une colonne de lavage à l'eau 52, pour en extraire l'acide, puis ils sont transférés dans une presse à vis 53 sur laquelle ils sont déshydratés avant d'être brûlés et gazéifiés dans un four à brûleur 54 pour fournir la vapeur, stockée dans le conteneur 55, destinée à être utilisée pour la furfuralisation.

La solution de pentoses contenant l'acide fort concentré est recueillie en tête du réacteur 48 et est envoyée par la conduite 56 dans un réacteur 57 de furfuralisation, après avoir été mise en température dans un échangeur de chaleur 58, et éventuellement additionnée d'un agent antimoussant. La colonne de furfuralisation 57 peut éventuellement contenir un garnissage de contact en matériau approprié. De la vapeur d'eau à 110 °C est introduite dans le bas de la colonne de furfuralisation 57, à contre-courant de la solution de pentoses à transformer en furfural, sous une pression de 1,3 bars. Plutôt que d'introduire la

vapeur directement dans la colonne 57, comme dans l'exemple de réalisation représenté à la fig. 2, dans l'installation représentée de façon schématique à la fig. 3, on fait recirculer la solution qui descend par gravité à la base de la colonne 57, à l'aide d'une pompe 59 dans un bouilleur à thermo-siphon 60 dans lequel une partie du liquide est vaporisée, la vapeur formée étant injectée par la conduite 61 à la base de la colonne 57.

La vapeur furfuralée sort en tête de colonne à 30% en poids de furfural. Après condensation et refroidissement dans le condenseur 62, le mélange eau-furfural se sépare en deux phases dans un séparateur 65:

- une phase à 95% en poids de furfural qui est envoyée, par la conduite 63, dans la colonne de déshydratation 64;
- une phase à 8% en poids de furfural qui est réinjectée à l'aide de la pompe 66 dans la colonne de furfuralisation 57.

De préférence, la phase à 95% en poids de furfural est soumise, avant d'être introduite dans la colonne de déshydratation 64, à un processus de neutralisation, de préférence à l'aide de $Na_2CO_3$, dans un réacteur 67 d'où elle est reprise dans la cuve 68.

L'acide fort concentré est soutiré à la base de la colonne de furfuralisation 57 en vue de son recyclage dans le réacteur d'hydrolyse 48. Toutefois, compte tenu de l'humidité résiduelle des matières végétales traitées par l'acide dans le réacteur 48 et de l'eau générée au cours de la réaction de furfuralisation, le titre de l'acide a tendance à être inférieur à sa composition initiale, à sa sortie de la colonne 57. Il est donc opportun de ramener l'acide à son titre initial avant de le recycler dans le réacteur 48. Une telle rectification est réalisée dans une colonne de distillation 69 dans laquelle l'acide dilué soutiré de la colonne 57 est amené par la conduite 70. On recueille:

- en tête de la colonne 69 l'eau résiduelle et les éventuelles substances volatiles entraînées par l'acide à sa sortie de la colonne 57 (telles que méthanol)
- en fond de la colonne 69, l'acide rectifié prêt à être recyclé dans le réacteur 48 par la conduite 71 (en passant par le réservoir de stockage 49 et la conduite 50).

La colonne de distillation 69 traite non seulement l'acide soutiré à sa sortie de la colonne de furfuralisation 57, mais avantageusement également l'acide extrait des résidus végétaux à leur sortie du réacteur 48, qui lui est amené par la conduite 73 en provenance du réservoir 72.

La phase à 95% en poids de furfural est introduite, par la conduite 74, dans la colonne de déshydratation 64 qui fonctionne sous un vide de 100 mm Hg, à 100 °C. La colonne 64 est une colonne de distillation à plateaux dans laquelle le furfural provenant de la colonne de furfuralisation, qui est un furfural technique à 95%, est déshydraté pour en éliminer l'eau présente en tant qu'impureté. Le furfural à 99% obtenu à la sortie de la colonne de distillation 64 est évacué de cette dernière à l'aide de la pompe 75, pour être envoyé dans la cuve de réception 76 puis, à l'aide de la pompe 77, dans la cuve de stockage de furfural anhydre 78.

L'eau séparée du furfural et contenant une faible quantité de furfural dissous, est évacuée en tête de la colonne de distillation 64 pour être envoyée dans un condenseur 79, puis un séparateur 80 d'où le furfural décanté est renvoyé dans la colonne 64 par la conduite 81, tandis que l'eau est évacuée vers un éjecteur 82 à partir duquel elle peut être envoyée dans le bouilleur à thermosiphon 60 où elle est vaporisée, puis recyclée dans la colonne de furfuralisation 57.

De même que les résidus végétaux de l'hydrolyse sont incinérés pour fournir de la vapeur pour l'étape de furfuralisation, rendant ainsi le procédé autonome énergétiquement, les impuretés soutirées au pied (83) de la colonne de furfuralisation 57 sont également envoyées à l'incinérateur 54 pour y être brûlées et fournir de la vapeur utilisable dans le procédé, et les impuretés issues de l'hydrolyse des matières végétales récupérées au pied (84) de la colonne 69 de régénération de l'acide fort concentré d'hydrolyse sont envoyées, après neutralisation dans le réacteur 85 et séparation dans le décanteur 86, à l'incinérateur 54 où elles sont également brûlées pour fournir de la vapeur au procédé.

On décrira ci-après, à titre d'exemple l'application de l'installation schématisée à la fig. 3, à la production de 5000 t/an de furfural, à partir de 35 000 t/an de rafles de maïs à 30% d'humidité (étant cependant entendu que le procédé conforme à l'invention s'applique avec les mêmes avantages à l'obtention de furfural à partir de matières végétales riches en pentosanes, à teneur en humidité moindre), le poids de matière sèche étant donc de l'ordre de 25 000 t/an et sa composition étant de:

- 32% de pentosanes
- 50% de cellulose
- 18% de lignine.

L'hydrolyseur 48 dont le volume est de 40 $m^3$ est alimenté en rafles de maïs à raison d'environ 3,12 t/heure de matière sèche et en acide chlorhydrique à 20% à 60 °C, à raison de 9,36 $m^3$/heure. Le débit de la solution d'hydrolysat à la sortie 56 de l'hydrolyseur 48 est de 6,55 $m^3$/heure et la concentration en pentoses de l'hydrolysat est de 150 g/litre.

La solution d'hydrolysat contenant 150 g/litre de pentoses entre dans la colonne de furfuralisation 57 dont le volume utile est de 14 $m^3$ à un débit de 6,55 $m^3$/heure. La vapeur est injectée à 110 °C, à une pression de 1,3 bars, à un débit de 1,5 t/heure. La vapeur furfuralée qui sort de la colonne 57 est condensée dans le condenseur 62 pour donner notamment un mélange eau-furfural à 95% en poids de furfural (et une concentration en pentoses de 8 g/litre). Dans ces conditions, le débit de la pompe de recirculation 59 dans le bouilleur 60 est de 27 $m^3$/heure.

La régénération de l'acide d'hydrolyse, l'acide chlorhydrique, à sa sortie de la colonne de furfuralisation 57 et préalablement à son recyclage dans

l'hydrolyseur 48, est effectuée dans la colonne de distillation 69, qui est de préférence une colonne à plateaux. La colonne 69 est alimentée par une solution d'acide chlorhydrique issue:

– de l'extraction des résidus végétaux à leur sortie de l'hydrolyseur 48, collectée dans le réservoir 72: 3,46 T/heure à 13% d'HCl
– de la furfuralisation: 8,56 T/heure à 17% d'HCl

soit, au total, 11,7 T/heure à 15,8% d'HCl en poids, à un débit d'alimentation de 12 T/heure à 15,8% d'HCl en poids. Le débit de soutirage de l'HCl régénéré en fond de colonne est de 9,3 T/heure d'HCl à 20% azéotropique.

Le débit de sortie de l'eau résiduelle en tête de colonne est de 2,42 T/heure et le débit d'impuretés en pied de colonne est de 0,3 T/heure.

Les pertes d'HCl sont de 1% par rapport à l'HCl d'alimentation, soit, sur une unité de 5000 T/an de furfural,

au niveau de la furfuralisation:
$1,4 \times 0,01 = 0,014$ T/heure d'HCl
au niveau du soutirage des impuretés:
0,3 T/heure $\times$ 0,2 = 0,06 T/heure d'HCl, soit 0,4 T/T de furfural.

Le mélange à 95% en poids de furfural est envoyé après décantation dans le décanteur 65 et neutralisation dans le réacteur 67, à un débit d'alimentation de 0,67 T/heure, dans une colonne de déshydratation 64 qui fonctionne sous un vide de 100 mm Hg, pour obtenir à la sortie de la colonne 64, un furfural à 99% en poids, à un débit de sortie de 0,65 T/heure, après avoir extrait 0,04 T/heure d'eau environ. On obtient le furfural à 99% à raison de 625 kg/heure.

La consommation totale de l'installation en vapeur d'eau est de 7 T/heure et la consommation totale d'eau de 185 m³/heure.

La combustion des 2,12 T/heure (en matière sèche) de résidus végétaux issus de l'hydrolyseur 48 – avec un PCI de 3400 kcal/kg – produit 7200 Th/h, soit 11 T/heure de vapeur, qui est donc fournie en un large excédent par rapport aux besoins de l'installation (environ 7 T/heure).

L'influence de la température sur la cinétique de la réaction d'hydrolyse des pentosanes en pentoses a été déterminée en étudiant l'évolution de la concentration en pentose dans le réacteur d'hydrolyse fermé, en fonction du temps pour plusieurs températures.

Les conditions de ces essais sont réunies dans le Tableau I ci-après:

Tableau 1

Essais d'hydrolyse de matières végétales riches en pentosanes en pentoses

Rafles utilisées: rafles de maïs séchées en Crib – composition: 11% d'humidité en poids
38% de pentosanes en poids
– densité: 160 kg/m³

| Essai N° | Température (°C) | Poids de rafles brutes dans l'hydrolyseur (kg) | Poids de matière sèche (kg) | Poids de pentosanes (kg) | Volume d'acide à 20% dans l'hydrolyseur (l) | Volume liquide total (l) | Concentration maximale en pentoses dans l'hydrolyseur (g/l) | Poids de matière sèche / Poids de liquide acide |
|---|---|---|---|---|---|---|---|---|
| 1 | 23 | 45,6 | 40,5 | 15,4 | 173 | 178 | 99 | 0,213 |
| 2 | 47 | 40,4 | 36 | 13,6 | 150 | 154,4 | 100 | 0,218 |
| 3 | 60 | 40 | 35,6 | 13,5 | 175 | 179,4 | 85 | 0,185 |

L'évolution de la concentration en pentoses dans l'hydrolyseur en fonction de la température, ressort des courbes de la fig. 4 dans laquelle:

- la courbe (1) montre l'évolution à 23 °C
- la courbe (2) montre l'évolution à 47 °C
- la courbe (3) montre l'évolution à 60 °C

Ces essais permettent la mise en évidence de deux phénomènes:

- l'apparition d'un équilibre pentosanes-pentoses qui ralentit la cinétique d'hydrolyse et qui empêche d'atteindre la concentration maximale en pentoses, dans le réacteur fermé,
- l'apparition d'une réaction de dégradation pour la température de 60 °C et une durée d'hydrolyse de 5 heures environ

et permettent de calculer l'enthalpie de la réaction d'hydrolyse:

$\Delta H = 15{,}5$ kcal/mol

L'étude de l'influence du temps de séjour de l'hydrolysat dans la colonne de furfuralisation sur le rendement en furfural a donné les résultats suivants, qui ont été traduits en courbes dans la fig. 5 annexée:

- le rendement en furfural croît en fonction du temps de séjour dans la colonne de furfuralisation. L'optimum est obtenu lorsqu'on adapte le temps de séjour à la cinétique d'obtention du furfural: cf. courbe (1) de la fig. 5 où ts = 2 heures;
- le rendement en furfural décroît pour des temps de séjour élevés (ts > 2 heures); les réactions parasites ne sont plus négligeables.

La courbe 2 donne le rendement molaire furfural + pentose obtenu par mole de pentose injecté et la courbe 3 donne le taux de pentoses non transformés en furfural. Ces courbes permettent de vérifier les excellents rendements du furfural que permet d'atteindre le procédé conforme à la présente invention.

Il résulte de la description qui précède que, quels que soient les modes de mise en œuvre, de réalisation et d'application adoptés, l'on obtient des procédés et appareillages pour l'obtention de furfural à partir de matières végétales, qui présentent par rapport aux procédés et appareillages visant au même but antérieurement connus, des avantages importants dont certains ont été mentionnés dans ce qui précède et dont d'autres avantages ressortiront de l'utilisation desdits procédés et appareillages.

## Revendications

1. Procédé de production de furfural à partir de matières végétales contenant des pentosanes, par hydrolyse de ces derniers en présence d'un acide fort et déshydratation subséquente, lequel procédé est caractérisé en ce qu'il est réalisé en deux étapes dont chacune est menée dans un réacteur séparé, en ce qu'au cours d'une première étape, on effectue, dans un premier réacteur, l'hydrolyse des pentosanes contenus dans la matière végétale, en présence d'acide chlorhydrique 5 à 6N à 20% en poids de HCL, l'hydrolyse étant réalisée en l'espace de 1 à 2 heures, par circulation à contre-courant liquide–solide, à la pression atmosphérique, à une température de 20 à 70 °C, la proportion d'acide chlorhydrique représentant 66 à 108% en poids des matières végétales sèches, pour obtenir une solution de pentoses, et en ce qu'au cours d'une deuxième étape, qui est réalisée dans un deuxième réacteur, ladite solution de pentoses est déshydratée pour obtenir du furfural, par action de la vapeur à une pression de 1 à 2 bars absolus et à une température inférieure ou égale à 110 °C, en milieu acide chlorhydrique à 20% en poids, ladite déshydratation étant réalisée par circulation à contre-courant de la solution de pentoses et de la vapeur de déshydratation, la solution de pentoses étant admise en tête du réacteur d'où elle s'écoule par gravité alors que la vapeur circule à contre-courant à partir du fond du réacteur, pour obtenir séparément, en tête du réacteur, le furfural formé, qui fournit, après décantation, une couche de furfural à 90% en poids exempt d'acide et en soutirage du réacteur, la solution d'acide chlorhydrique, qui est récupérée pour être recyclée dans une nouvelle mise en œuvre du procédé, dans l'étape de l'hydrolyse.

2. Procédé selon la revendication 1, caractérisé en ce que dans le cas où l'on souhaite provoquer la dégradation de la cellulose des matières végétales jusqu'au stade des sucres, on prolonge le temps de contact entre les matières végétales et ledit acide jusqu'à 4 à 12 heures pour provoquer ladite dégradation et récupérer les sucres résultant de cette dernière.

3. Procédé selon l'une quelconque des revendications 1 et 2, caractérisé en ce que le processus d'hydrolyse est accéléré en soumettant le milieu réactionnel à agitation, par recirculation de la solution acide de pentoses obtenue au cours de la première étape, dans le milieu réactionnel constitué par les matières végétales et l'acide fort concentré.

4. Procédé selon l'une quelconque des revendications 1 à 3, caractérisé en ce que la vapeur de déshydratation mise en œuvre au cours de la deuxième étape du procédé est à une température de 100 à 110 °C environ.

5. Procédé selon la revendication 1, caractérisé en ce que la solution de pentoses à déshydrater et la vapeur de déshydratation sont introduites en continu dans l'étape de déshydratation.

6. Procédé selon la revendication 1, caractérisé en ce que la valeur de déshydratation introduite dans l'étape de déshydratation, est obtenue par combustion et gazéification des résidus végétaux issus de l'étape d'hydrolyse.

7. Procédé selon la revendication 1, caractérisé en ce que la vapeur de déshydratation introduite dans l'étape de déshydratation, est obtenue par vaporisation d'une partie de la solution de pentoses introduite dans l'étape de déshydratation.

8. Procédé selon l'une quelconque des revendications 1 à 7, caractérisé en ce que la solution de pentoses à déshydrater est additionnée d'un agent anti-moussant.

9. Procédé selon l'une quelconque des revendications 1 à 8, caractérisé en ce que l'acide récupéré à la fin de l'étape de déshydratation est soumis, préalablement à son recyclage, à un processus de distillation pour le ramener à sa composition azéotropique de 20% en poids.

10. Procédé selon l'une quelconque des revendications 1 à 9, caractérisé en ce que les résidus résultant de l'hydrolyse des matières végétales sont séparés par chauffage de l'acide concentré utilisé pour l'hydrolyse, pour être récupérés et valorisés.

11. Procédé selon l'une quelconque des revendications 1 à 10, caractérisé en ce que le furfural obtenu est soumis à un processus de purification, pour obtenir du furfural pur.

12. Procédé selon la revendication 11, caractérisé en ce que le furfural obtenu à l'issue de l'étape de déshydratation est purifié par distillation, par application d'un vide de l'ordre de 0,1 à 0,3 bar absolu, à une température pouvant atteindre 100–110 °C.

13. Appareillage pour la production de furfural en mettant en œuvre le procédé selon l'une quelconque des revendications 1 à 12, lequel appareillage est caractérisé en ce qu'il comprend en combinaison: – au moins un premier réacteur associé à un dispositif d'amenée des matières végétales et à un réservoir de stockage d'acide fort concentré auquel il est relié par une conduite d'alimentation qui amène l'acide fort concentré, dans le premier réacteur, à contre-courant de la matière végétale; – au moins un deuxième réacteur à la partie supérieure duquel débouche une conduite d'amenée de la solution de pentoses contenant l'acide fort concentré en provenance d'au moins un premier réacteur, et qui comporte à sa partie inférieure des moyens d'introduction de vapeur d'eau à une température de l'ordre de 100 à 110 °C pour assurer la circulation de la vapeur d'eau à contre-courant de la solution de pentosanes susdite;
– une conduite d'évacuation de l'acide concentré sensiblement à la base dudit deuxième réacteur, laquelle conduite débouche dans la conduite d'alimentation en acide d'au moins un premier réacteur et qui recycle ledit acide dans ce dernier, et une conduite, raccordée à la tête dudit deuxième réacteur, d'évacuation des vapeurs furfuralées exemptes d'acide vers un condenseur et un décanteur d'où le furfural est envoyé par des moyens appropriés à un réservoir de stockage.

14. Appareillage selon la revendication 13, caractérisé en ce qu'au moins un premier réacteur est associé à des moyens d'agitation du mélange réactionnel qu'il contient et qui est essentiellement constitué par les matières végétales et l'acide fort concentré, lesquels sont essentiellement constitués par des moyens de recirculation de la solution acide de pentoses, qui recyclent cette dernière dans au moins un premier réacteur.

15. Appareillage selon l'une quelconque des revendications 13 et 14, caractérisé en ce que le(s) deuxième(s) réacteur(s) est (ou sont) rempli(s) d'un garnissage pour améliorer la distribution et le contact des réactifs dans le(s)dit(s) deuxième(s) réacteur(s).

16. Appareillage selon l'une quelconque des revendications 13 à 15, caractérisé en ce que le(s) deuxième(s) réacteur(s) est (ou sont) entouré(s) de moyens de réchauffage dudit (ou desdits) réacteur(s) tels que serpentins de réchauffage à la vapeur.

17. Appareillage selon l'une quelconque des revendications 13 à 16, caractérisé en ce que l'eau condensée récupérée à la sortie du condenseur et du décanteur est envoyée à un bouilleur, d'où elle est recyclée, après vaporisation, dans la conduite d'introduction de vapeur d'eau dans au moins un deuxième réacteur.

18. Appareillage selon la revendication 17, caractérisé en ce que le bouilleur est un bouilleur fonctionnant en thermo-siphon.

19. Appareillage selon l'une quelconque des revendications 13 à 18, caractérisé en ce qu'il comporte, en outre, une installation de purification de furfural provenant du réservoir de stockage susdit, dans laquelle le furfural est débarrassé de l'eau qu'il contient et qui est reliée à un réservoir de stockage de furfural anhydre.

20. Appareillage selon la revendication 19, caractérisé en ce que l'installation de purification susdite comprend une colonne de distillation mise sous un vide de l'ordre de 0,1 à 0,3 bar et maintenue à une température pouvant atteindre 100 à 110 °C.

21. Appareillage selon l'une quelconque des revendications 13 à 20, caractérisé en ce que l'acide de déshydratation évacué du ou des deuxième(s) réacteur(s) est envoyé, préalablement à son recyclage dans au moins un premier réacteur, dans une colonne de distillation pour en extraire l'eau (notamment l'eau générée au cours de la réaction) et le ramener à sa composition initiale.

22. Appareillage selon l'une quelconque des revendications 13 à 21, caractérisé en ce qu'il comprend, en outre, un condenseur-évaporateur dans lequel les sucres, et en particulier le glucose, obtenus par attaque acide de la cellulose des matières végétales, sont séparés de l'acide évacué d'au moins un deuxième réacteur, pour être récupérés à la sortie dudit condenseur-évaporateur, préalablement au recyclage de l'acide dans au moins un premier réacteur.

**Claims**

1. Process for the production of furfural from plant materials containing pentosans, by hydrolysis of the latter in the presence of a strong acid and subsequent dehydration, which process is characterized in that it is carried out in two steps each of which is conducted in a separate reactor, in that in the course of a first step, the hydrolysis of the pentosans contained in the plant material is carried out in the presence of 5 to 6N hydrochloric acid with 20% by weight of HCl, the hydrolysis being effected in the space of 1 to 2 hours, by liquid-solid counter-current flow, at atmospheric

pressure, the temperature of 20 to 70 °C, the proportion of hydrochloric acid representing 66 to 108% by weight of the plant materials to obtain a solution of pentoses, and in that in the course of a second step, which is carried out in a second reactor, said solution of pentoses is dehydrated to obtain furfural, by the action of steam at the pressure of 1 to 2 bars absolute and at a temperature below or equal to 110 °C, in a medium with 20% by weight of hydrochloric acid, said dehydration being effected by counter-current flow of the solution of pentoses and of the dehydration steam, the solution of pentoses being admitted at the top of the reactor whence it flows by gravity whilst the steam flows in counter-current from the bottom of the reactor, to obtain separately, at the head of the reactor, the furfural formed, which provides, after decantation, a layer of furfural with 90% by weight free of acid and by drawing off from the reactor, the hydrochloric acid solution, which is recovered to be recirculated in a further application of the process, in the hydrolysis step.

2. Process according to claim 1, characterized in that in the case where it is desired to cause the degradation of the cellulose of the plant materials as far as the stage of sugars, the contact time between the plant materials and said acid is prolonged up to 4 to 12 hours to cause said degradation and recover the sugars resulting from the latter.

3. Process according to any one of claims 1 and 2, characterized in that the hydrolysis process is accelerated by subjecting the reaction medium to stirring, by recirculation of the acid solution of pentoses obtained in the course of the first step, into the reaction medium constituted by the plant materials and the concentrated strong acid.

4. Process according to any one of claims 1 to 3, characterized in that the dehydration steam employed in the course of the second step of the process is at a temperature of about 100 to 110 °C.

5. Process according to claim 1, characterized in that the pentose solution for dehydration and the dehydration steam are introduced continuously in the dehydration step.

6. Process according to claim 1, characterized in that the dehydration steam introduced in the dehydration step is obtained by combustion and gasification of the plant residues emerging from the hydrolysis step.

7. Process according to claim 1, characterized in that the dehydration vapor introduced in the dehydration step, is obtained by vaporization of a part of the pentose solution introduced in the dehydration step.

8. Process according to any one of claims 1 to 7, characterized in that the pentose solution to be dehydrated is supplemented with an anti-foaming agent.

9. Process according to any one of claims 1 to 8, characterized in that the acid recovered at the end of the dehydration step is subjected, prior to its recirculation, to a distillation process to restore it to its azeotropic composition of 20% by weight.

10. Process according to any one of claims 1 to 9, characterized in that the residues resulting from the hydrolysis of the plant materials are separated by heating the concentrated acid used for the hydrolysis, to be recovered and valorized.

11. Process according to any one of claims 1 to 10, characterized in that the furfural obtained is subjected to a purification process, to obtain pure furfural.

12. Process according to claim 11, characterized in that furfural obtained at the end of the dehydration step is purified by distillation, by application of a vacuum of the order of 0.1 to 0.3 bars absolute, at a temperature which can reach 100 to 110 °C.

13. Apparatus for the production of furfural by employing the process according to any one of claims 1 to 12, which apparatus is characterized in that it comprises in combination: – at least one first reactor associated with an input device for the plant materials and with a storage tank for concentrated strong acid to which it is connected by a supply pipe which leads the concentrated strong acid, into the first reactor, in counter-current to the plant material; – at least one second reactor at the upper part of which opens a pipe for introducing the solution of pentoses containing the concentrated strong acid coming from at least one first reactor, and which comprises at its lower part means for the introduction of steam at a temperature of the order of 100 to 110 °C to ensure the circulation of the steam in counter-current with the above-mentioned solution of pentosans; – a pipe for removing the concentrated acid substantially at the base of said second reactor, which pipe opens into the acid supply pipe of at least one first reactor and which recycle said acid into the latter, and a pipe, connected to the top of said second reactor, for removing furfural vapor free of acid to a condenser and a decanter whence the furfural is sent by suitable means to a storage tank.

14. Apparatus according to claim 13, characterized in that at least one first reactor is associated with stirring means for the reaction mixture that it contains and which is essentially constituted by plant materials and concentrated strong acid, which are essentially constituted by recirculation means for the acid solution of pentose, which recycle the latter into at least one first reactor.

15. Apparatus according to any of claims 13 and 14, characterized in that said second reactor(s) is (or are) filled with a lining to improve the distribution and the contact of the reagents in said second reactor(s).

16. Apparatus according to any one of claims 13 to 15, characterized in that the second reactor(s) is (or are) surrounded by reheating means for said reactor(s) such as steam-heating coils.

17. Apparatus according to any one of claims 13 to 16, characterized in that the condensed water recovered at the outlet from the condenser and from the decanter is sent to a boiler, whence it is recycled, after vaporization, into the pipe for introducing steam into at least one second reactor.

18. Apparatus according to claim 17, characterized in that the boiler is a boiler operating by thermo-siphon.

19. Apparatus according to any one of claims 13 to 18, characterized in that it comprises in addition, an installation for the purification of furfural coming form the abovesaid storage tank, in which the furfural is freed from water that it contains and which is connected to a storage tank for anhydrous furfural.

20. Apparatus according to claim 19, characterized in that the abovesaid purification installation comprises a distillation column placed under a vacuum of the order of 0.1 to 0.3 bar and kept at a temperature which can reach 100 to 110 °C.

21. Apparatus according to any one of claims 13 to 20, characterized in that the dehydration acid removed from the one or more second reactor(s) is sent, prior to its recycling into at least one first reactor, into a distillation column to extract the water therefrom (particularly the water generated in the course of the reaction) and to restore it to its initial composition.

22. Apparatus according to any one of claims 13 to 21, characterized in that it comprises, in addition, a condenser-evaporator in which the sugars, and in particular the glucose, obtained by acid attack of the cellulose of the plant materials, are separated from the acid removed from at least one second reactor, to be recovered at the outlet of said condenser-evaporator, prior to the recycling of the acid into at least one first reactor.

**Patentansprüche**

1. Verfahren zur Herstellung von Furfural, ausgehend von Pentosane enthaltenden pflanzlichen Materialien, durch Hydrolyse der letzteren in Anwesenheit einer starken Säure und anschliessendes Entwässern, dadurch gekennzeichnet, dass das Verfahren in zwei Stufen durchgeführt wird, von denen jede in einem getrennten Reaktor durchgeführt wird, wobei im Verlauf einer ersten Stufe in einem ersten Reaktor die in dem pflanzlichen Material enthaltenen Pentosane in Anwesenheit von 5 bis 6 n Chlorwasserstoffsäure mit 20 Gew.-% HCl hydrolysiert werden, die Hydrolyse in einem Zeitraum von 1 bis 2 h durch Flüssigkeits-Feststoff-Zirkulation im Gegenstrom bei Atmosphärendruck bei einer Temperatur von 20 bis 70 °C durchgeführt wird, wobei der Chlorwasserstoffanteil 66 bis 108 Gew.-% der trockenen, pflanzlichen Materialien beträgt, um eine Lösung von Pentosen zu erhalten, und im Verlauf einer zweiten Stufe, die in einem zweiten Reaktor durchgeführt wird, die Lösung der Pentosen zur Erzielung von Furfural durch Einwirken von Dampf mit einem Druck von 1 bis 2 bar absolut und bei einer Temperatur unter oder gleich 110 °C in einem Chlorwasserstoffsäuremilieu von 20 Gew.-% entwässert wird, wobei die Entwässerung durch Zirkulieren der Lösung der Pentosen und des Entwässerungsdampfes im Gegenstrom durchgeführt wird, wobei die Lösung der Pentosen am Kopf des Reaktors eingeführt wird, von wo sie durch die Schwerkraft abströmt, wohingegen der Dampf im Gegenstrom ausgehend vom Boden des Reaktors zirkuliert, um getrennt im Kopf des Reaktors das gebildete Furfural, das nach dem Dekantieren eine 90 Gew.-% Furfuralschicht frei von Säure ergibt, und am Abzug des Reaktors die Chlorwasserstoffsäurelösung zu erhalten, die zur Recyclisierung in einen neuen Ansatz des Verfahrens in die Hydrolysestufe zurückgewonnen wird.

2. Verfahren nach Anspruch 1, dadurch gekennzeichnet, dass man, falls die Zersetzung der Cellulose der pflanzlichen Materialien bis zum Zuckerstadium gewünscht wird, die Kontaktzeit zwischen den pflanzlichen Materialien und der Säure bis auf 4 bis 12 h verlängert, um die Zersetzung hervorzurufen und die aus letzterer resultierenden Zucker zu gewinnen.

3. Verfahren nach einem der Ansprüche 1 und 2, dadurch gekennzeichnet, dass das Hydrolyseverfahren durch Bewegen des Reaktionsmediums durch Rezirkulieren der sauren, im Verlauf der ersten Stufe erhaltenen Pentosenlösung in das Reaktionsmedium, das aus den pflanzlichen Materialien und der konzentrierten starken Säure besteht, beschleunigt wird.

4. Verfahren nach einem der Ansprüche 1 bis 3, dadurch gekennzeichnet, dass der im Verlauf der zweiten Stufe des Verfahrens eingesetzte Entwässerungsdampf eine Temperatur von etwa 100 bis 110 °C aufweist.

5. Verfahren nach Anspruch 1, dadurch gekennzeichnet, dass die zu entwässernde Pentosenlösung und der Entwässerungsdampf kontinuierlich in die Entwässerungsstufe eingeführt werden.

6. Verfahren nach Anspruch 1, dadurch gekennzeichnet, dass der in die Entwässerungsstufe eingeführte Entwässerungsdampf erhalten wird durch Verbrennen und Vergasen der in der Hydrolysestufe erhaltenen pflanzlichen Rückstände.

7. Verfahren nach Anspruch 1, dadurch gekennzeichnet, dass der in die Entwässerungsstufe eingeführte Entwässerungsdampf durch Verdampfen eines Teils der in die Entwässerungsstufe eingeführten Pentosenlösung erhalten wird.

8. Verfahren nach einem der Ansprüche 1 bis 7, dadurch gekennzeichnet, dass die zu entwässernde Pentosenlösung mit einem Antischaummittel versetzt wird.

9. Verfahren nach einem der Ansprüche 1 bis 8, dadurch gekennzeichnet, dass die am Ende der Entwässerungsstufe wiedergewonnene Säure vor ihrer Recyclisierung einem Destillationsverfahren unterzogen wird, um sie auf ihre azeotrope Zusammensetzung von 20 Gew.-% zurückzuführen.

10. Verfahren nach einem der Ansprüche 1 bis 9, dadurch gekennzeichnet, dass die aus der Hydrolyse der pflanzlichen Materialien resultierenden Rückstände durch Erwärmen der für die Hydrolyse verwendeten konzentrierten Säure abgetrennt werden, um wiedergewonnen und verwertet zu werden.

11. Verfahren nach einem der Ansprüche 1 bis 10, dadurch gekennzeichnet, dass das erhaltene Furfural einem Reinigungsverfahren zur Erzielung von reinem Furfural unterzogen wird.

12. Verfahren nach Anspruch 11, dadurch gekennzeichnet, dass das am Ausgang der Entwässerungsstufe erhaltene Furfural durch Destillation gereinigt wird, durch Anwendung eines Vakuums in der Grössenordnung von 0,1 bid 0,3 bar absolut, bei einer Temperatur, die 100–110 °C erreichen kann.

13. Vorrichtung zur Herstellung von Furfural unter Anwendung des Verfahrens gemäss einem der Ansprüche 1 bis 12, dadurch gekennzeichnet, dass die Vorrichtung eine Kombination enthält von: – mindestens einem ersten Reaktor, der verbunden ist mit einer Einrichtung zur Zufuhr der pflanzlichen Materialien und einem Speicherbehälter für die konzentrierte starke Säure, mit dem er über eine Bespeisungsleitung verbunden ist, die die konzentrierte starke Säure in den ersten Reaktor im Gegenstrom zum pflanzlichen Material führt; – mindestens einem zweiten Reaktor, in dessen oberes Teil eine Zufuhrleitung für die Pentosenlösung, die die konzentrierte starke Säure enthält, von mindestens einem ersten Reaktor her mündet und der an seinem unteren Teil Einrichtungen zur Einführung von Wasserdampf mit einer Temperatur in der Grössenordnung von 100–110 °C enthält, um die Zirkulation des Wasserdampfes im Gegenstrom zu der vorstehend genannten Lösung von Pentosanen sicherzustellen; – einer Entleerungsleitung für die konzentrierte Säure, ungefähr an der Basis des zweiten Reaktors, wobei diese Leitung in eine Bespeisungsleitung für Säure für mindestens einen ersten Reaktor mündet und die die Säure in den letzteren recyclisiert, und einer Leitung, die mit dem Kopf des zweiten Reaktors verbunden ist, zur Entleerung der Furfural enthaltenden von säurefreien Dämpfe zu einem Kondensator und einer Dekantiervorrichtung hin, von wo das Furfural durch geeignete Einrichtungen zu einem Speicherbehälter geführt wird.

14. Vorrichtung nach Anspruch 13, dadurch gekennzeichnet, dass mindestens ein erster Reaktor mit Einrichtungen zum Bewegen des enthaltenen Reaktionsgemischs, das im wesentlichen aus den pflanzlichen Materialien und der konzentrierten starken Säure gebildet wird, versehen ist, wobei diese im wesentlichen aus Rezirkulierungseinrichtungen für die saure Pentosenlösung bestehen, die letztere in mindestens einen ersten Reaktor recyclisieren.

15. Vorrichtung nach einem der Ansprüche 13 und 14, dadurch gekennzeichnet, dass der bzw. die zweite(n) Reaktor(en) mit einem Füllmaterial gefüllt ist (oder sind), um die Verteilung und den Kontakt der Reaktionskomponenten in dem bzw. den genannten zweiten Reaktor(en) zu verbessern.

16. Vorrichtung gemäss einem der Ansprüche 13 bis 15, dadurch gekennzeichnet, dass der bzw. die zweite(n) Reaktor(en) von Einrichtungen zur Wiedererwärmung des (oder der) Reaktors bzw. Reaktoren umgeben ist (oder sind), wie Dampf-Heizschlangen.

17. Vorrichtung gemäss einem der Ansprüche 13 bis 16, dadurch gekennzeichnet, dass das am Ausgang des Kondensators und der Dekantiervorrichtung wiedergewonnene Kondenswasser zu einem Kocher geführt wird, von wo es nach dem Verdampfen in die Zufuhrleitung für Wasserdampf in mindestens einen zweiten Reaktor recyclisiert wird.

18. Vorrichtung nach Anspruch 17, dadurch gekennzeichnet, dass der Kocher ein als Thermo-Siphon funktionierender Kocher ist.

19. Vorrichtung nach einem der Ansprüche 13 bis 18, dadurch gekennzeichnet, dass sie ausserdem eine Einrichtung zur Reinigung des aus dem vorstehend genannten Speicherbehälter kommenden Furfurals aufweist, worin das Furfural von dem es enthaltenden Wasser befreit wird, und die mit einem Speicherbehälter für wasserfreies Furfural verbunden ist.

20. Vorrichtung nach Anspruch 19, dadurch gekennzeichnet, dass die vorstehend genannte Reinigungseinrichtung eine Destillationskolonne umfasst, die unter einem Vakuum in der Grössenordnung von 0,1 bis 0,3 bar gehalten wird und bei einer Temperatur gehalten wird, die 100 bis 110 °C erreichen kann.

21. Vorrichtung nach einem der Ansprüche 13 bis 20, dadurch gekennzeichnet, dass die aus dem oder den zweiten Reaktor(en) abgezogene Entwässerungssäure vor ihrem Recyclisieren in mindestens einen ersten Reaktor in eine Destillationskolonne geschickt wird, um daraus das Wasser (insbesondere das im Verlauf der Reaktion gebildete Wasser) zu extrahieren und sie auf ihre ursprüngliche Zusammensetzung zurückzuführen.

22. Vorrichtung nach einem der Ansprüche 13 bis 21, dadurch gekennzeichnet, dass sie ausserdem einen Kondensator-Verdampfer enthält, in dem die durch Angriff der Cellulose der pflanzlichen Materialien mit Säure erhaltenen Zucker, und insbesondere die Glucose, von der aus mindestens einem zweiten Reaktor abgezogenen Säure abgetrennt werden, um am Ausgang des Kondensator-Verdampfers, vor dem Recyclisieren der Säure in mindestens einen ersten Reaktor, gewonnen zu werden.

Fig. 1

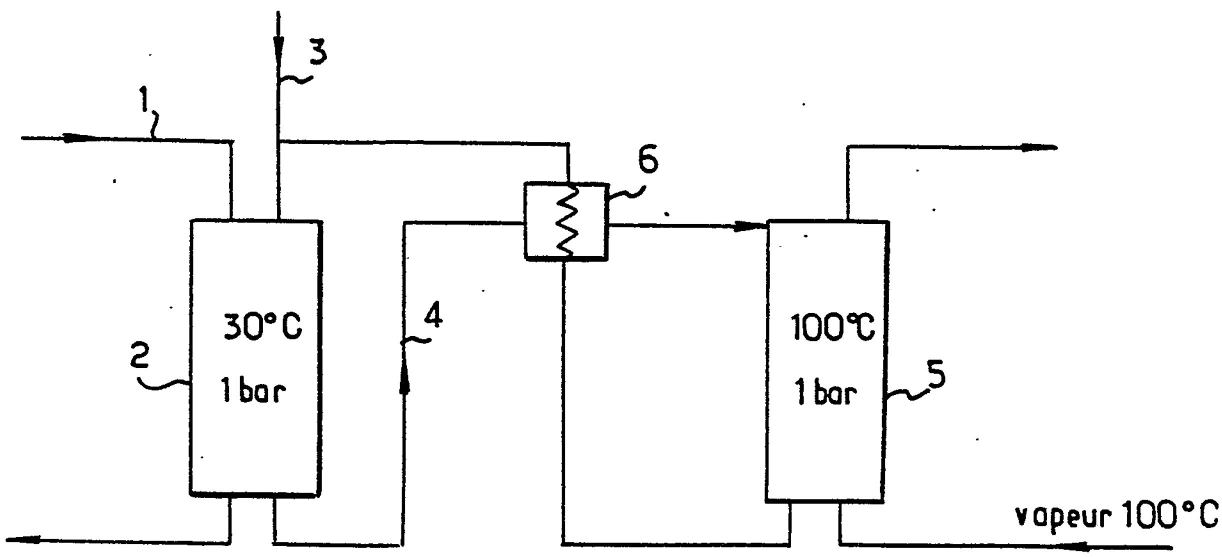

0 033 323

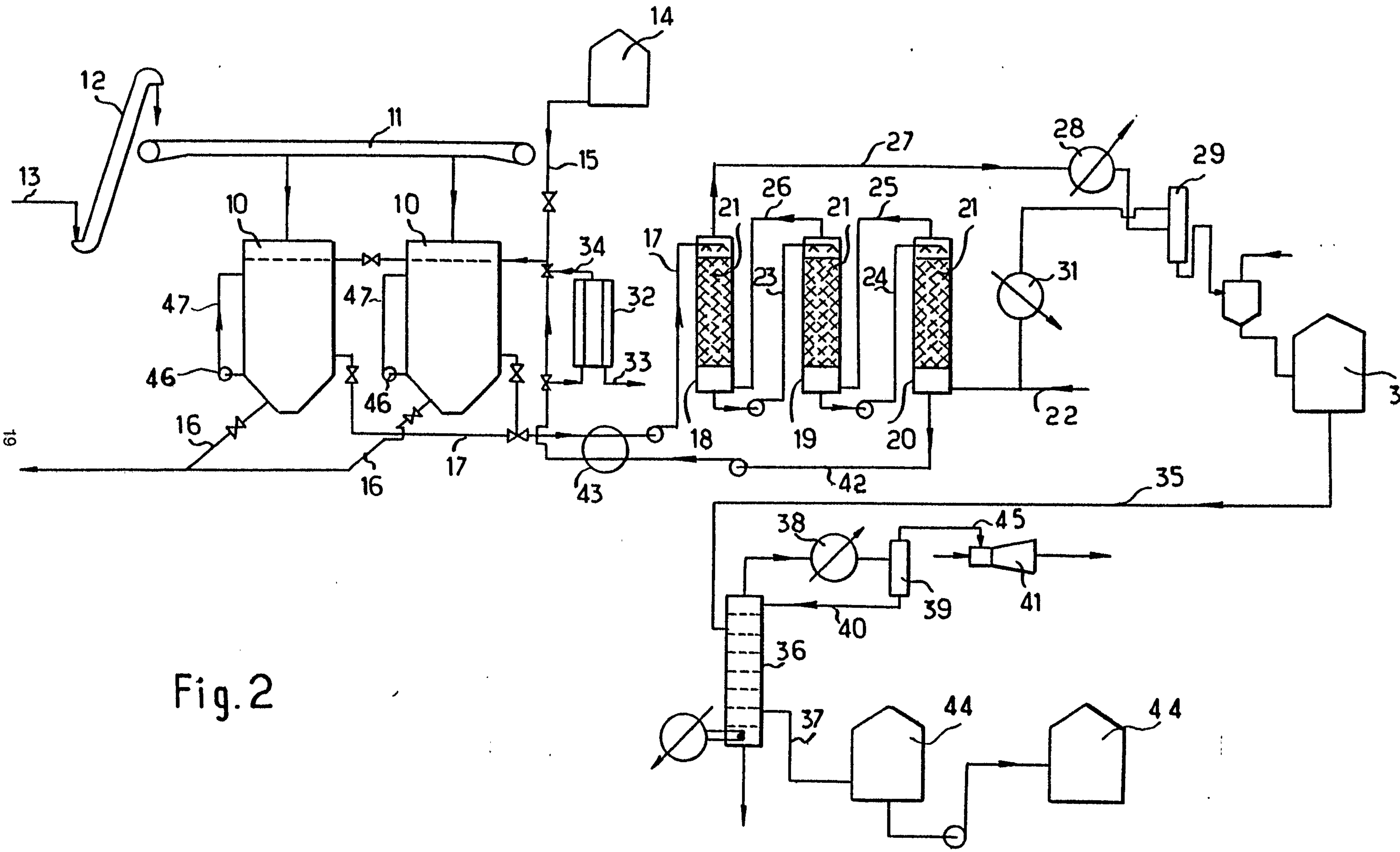


Fig. 2

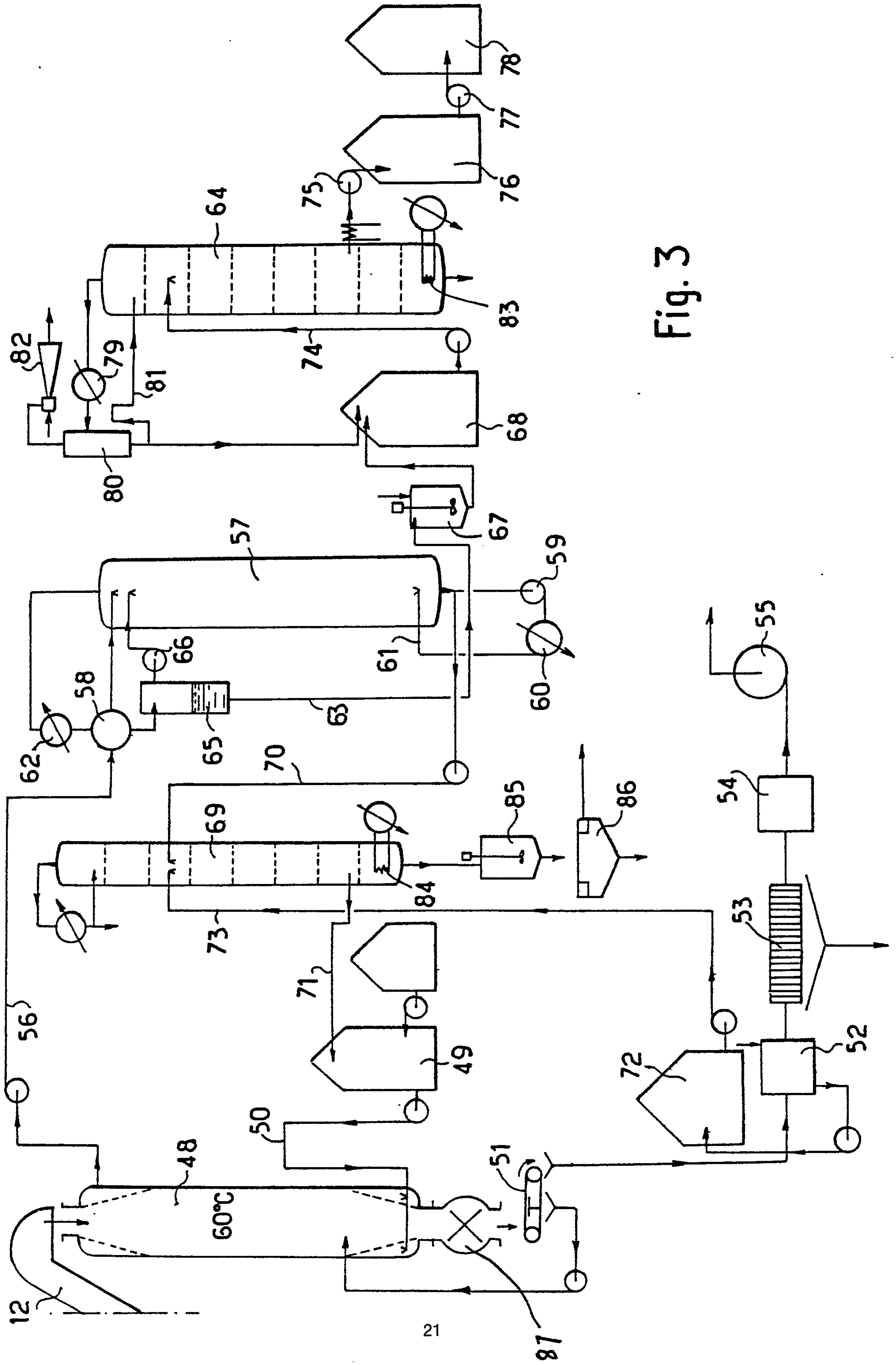
12
48
60°C
87
51
50
49
56
71
73
72
52
53
54
55
69
84
85
86
70
62
58
65
63
66
61
60
59
57
67
80
68
74
81
79
82
83
64
75
76
77
78

Fig. 3

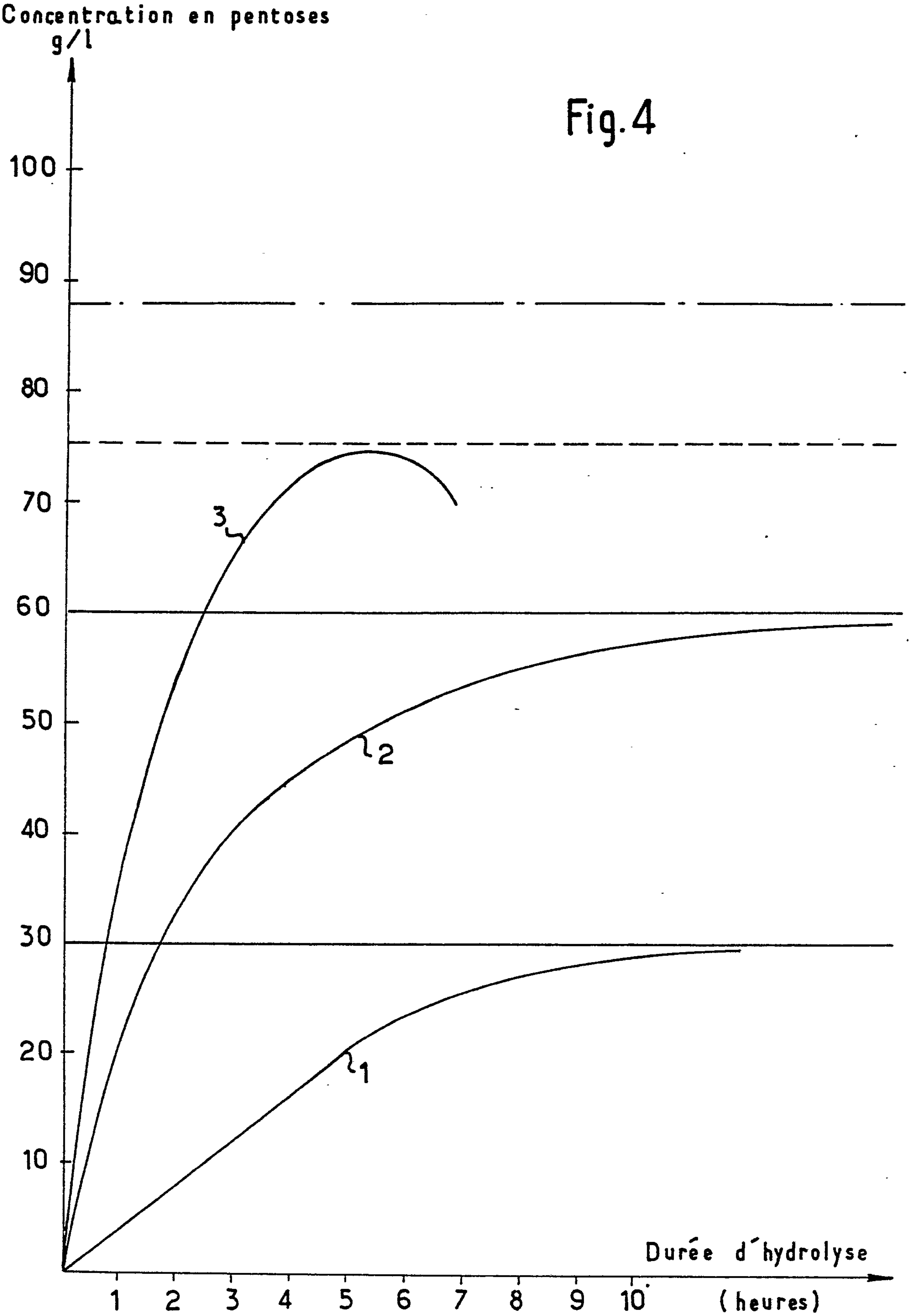
Concentration en pentoses
g/l
100
90
80
70
60
50
40
30
20
10
3
2
1
Durée d'hydrolyse
1
2
3
4
5
6
7
8
9
10
(heures)

Fig.4

Fig. 5

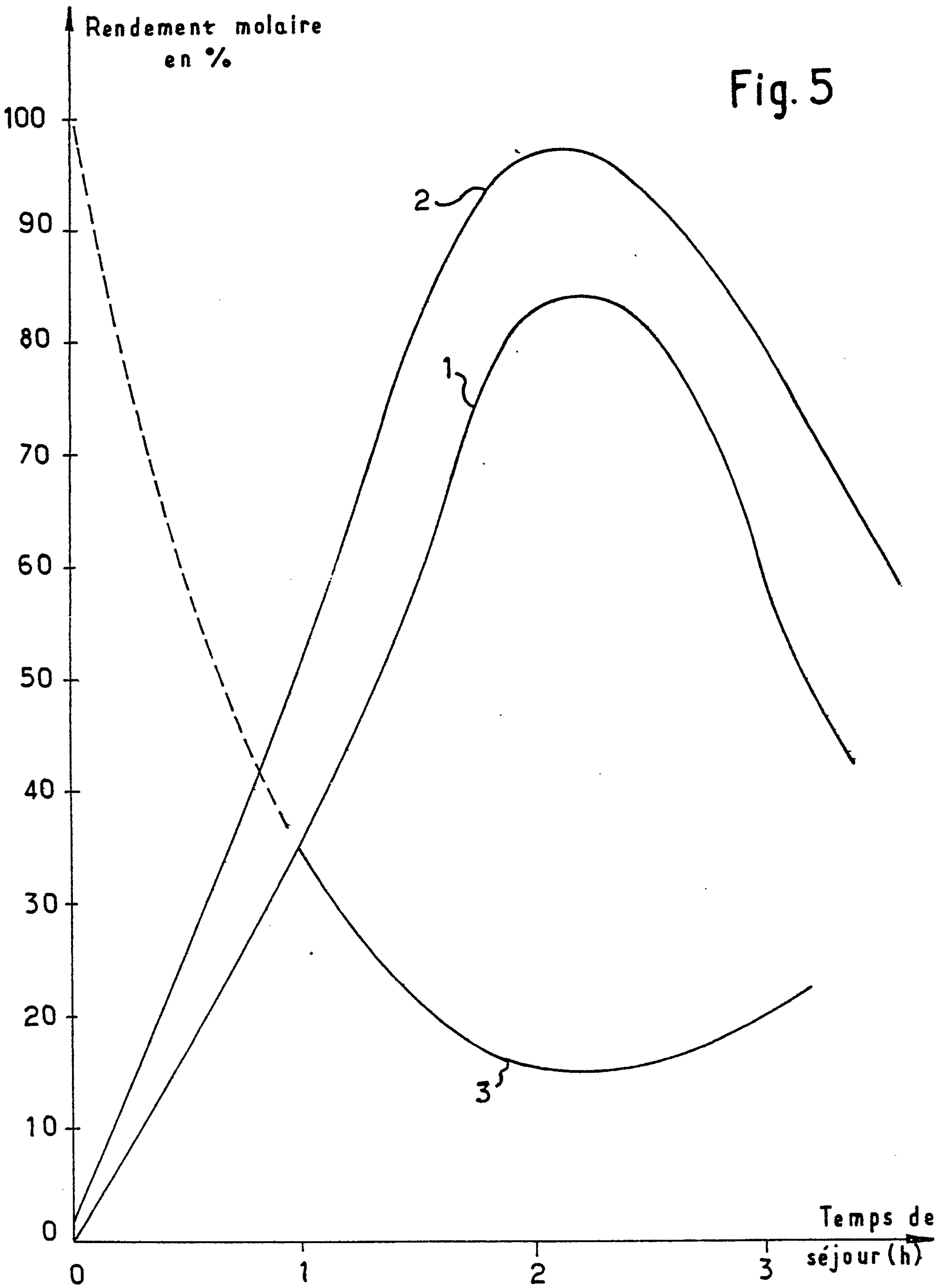
Rendement molaire
en %
100
90
80
70
60
50
40
30
20
10
0
2
1
3
Temps de
séjour (h)
0
1
2
3